# EUROPÄISCHE PATENTANMELDUNG

(11) **EP 0 563 767 A1**
(43) Veröffentlichungstag der Anmeldung: **06.10.1993**
(21) Anmeldenummer: 93104816.9
(22) Anmeldetag: 24.03.1993
(51) Int. Cl.: A61F 2/18

(54) **Gehörknöchelprothese**

(30) Priorität: 28.03.1992 DE 4210235
(71) Anmelder: Kurz, Heinz, D-72144 Dusslingen (DE)
(72) Erfinder: Kurz, Heinz, D-72144 Dusslingen (DE)
(74) Vertreter: Möbus, Rudolf, Dipl.-Ing.

(57) **Zusammenfassung**

Die Gehörknöchelprothese weist an mindestens einem der Enden eines aus Feingold gefertigten Stieles (21) einen Koppelungskörper (23) aus reinem Titan auf, der durch seine rauhe, aber körperverträgliche Oxidoberfläche eine besonders gute Verankerung auf einem Knochen- oder Knorpelteil gewährleistet.

## Beschreibung

Die Erfindung betrifft eine Gehörknöchelprothese zur Schallübertragung im Mittelohr, mit einem aus Feingold gefertigten Stiel.

Bisher aus Feingold gefertigte und implantierte Gehörknöchelprothesen haben sich hinsichtlich der Verträglichkeit und ihrer Schallübertragungseigenschaft hervorragend bewährt. Werden diese Prothesen jedoch direkt am Trommelfell eines Patienten angelegt, bleibt als wesentlicher Nachteil die Instabilität des Trommelfells bestehen, so daß die Prothese ihre Lage am Trommelfell ändern und sogar das Trommelfell durchwandern kann. Man ist daher bestrebt, die Gehörknöchelprothesen trommelfellseitig an dem häufig noch vorhandenen Hammergriff zu verankern. Hier erschwert jedoch die Materialbeschaffenheit einer Edelstahlprothese deren Befestigung.

Der Erfindung liegt die Aufgabe zugrunde, eine Gehörknöchelprothese der eingangs genannten Art so auszubilden, daß mit ihr eine gute Verankerung an den Gehörorganen erreicht wird.

Die gestellte Aufgabe wird mit der Gehörknöchelprothese erfindungsgemäß dadurch gelöst, daß an mindestens einem der Stielenden der Prothese ein aus reinem Titan gefertigter Koppelungskörper angeschlossen ist.

Teile aus reinem Titan überziehen sich unter dem Einfluß von Sauerstoff mit einer relativ rauhen Oxidschicht. Es hat sich herausgestellt, daß diese Titanoxidschicht sehr gut körperverträglich ist, also bei Berührung mit der Haut und mit Knochenteilen nicht zu Entzündungen reizt. Die rauhe Oberfläche der Titanoxidschicht verleiht dem Prothesenteil einen guten Sitz auf einem Knochen- oder Knorpelteil und gibt der Knochensubstanz oder Knorpelsubstanz die Möglichkeit, in die Vertiefungen der Oberfläche des Prothesenteiles einzuwachsen und dadurch die Verbindung mit der Prothese zusätzlich zu sichern. Durch besondere Schweißverfahren läßt sich eine gute und feste Verbindung zwischen dem aus Feingold gefertigten Teil und einem aus reinem Titan gefertigten Teil der Prothese schaffen.

Die Form der Gehörknöchelprothese und insbesondere ihrer an den Enden des Prothesenstieles angeordneten Teile kann unterschiedlich gestaltet sein, je nachdem, in welchem Umfange sie die Gehörknöchel ersetzen muß. Bei einer Vollprothese zwischen dem Trommelfell und der Fußplatte des Steigbügels kann vorteilhafterweise am einen Ende des drahtförmigen Feingoldstieles ein an einen Hammergriffrest am Trommelfell ansetzbares, aus reinem Titan gefertigtes Hakenteil vorgesehen sein, und am anderen Ende des Feingoldstieles kann die Vollprothese einen ebenfalls aus reinem Titan gefertigten geschlitzten Glockenkörper zur Anlage gegen die Fußplatte aufweisen. In Fällen, in welchen der Steigbügel noch weitgehend erhalten ist, kann am Ende des Feingoldstieles auch ein ebenfalls aus Feingold gefertigter geschlitzter Glockenkörper angebracht sein, während am anderen Ende des Feingoldstieles eine geschlitzte Manschette aus reinem Titan zum Ansatz am Amboßschenkel oder am Hammergriff angeordnet sein kann. Zur sicheren Verankerung der Gehörknöchelprothese bei der Operation kann der am einen Ende des Prothesenstieles angeordnete Glockenkörper zweckmäßig kreuzweise geschlitzt sein und dadurch eine Veränderung des Glockenranddurchmessers erleichtern. Auch die aus reinem Titan gefertigte Manschette kann vorteilhafterweise mehrfach geschlitzt sein und vorzugsweise aus einer längsgeschlitzten Titanbuchse bestehen, die zur Verbesserung ihrer Verformbarkeit an mindestens einer Stelle zusätzlich einen Teilquerschlitz aufweist. Vorteilhafterweise kann auch der aus Feingold gefertigte Stiel zur Anpassung an unterschiedlich gelegene Verankerungsstellen mit mindestens einer Abwinkelung, Ausbiegung oder Abkröpfung versehen sein.

Nachfolgend werden drei Ausführungsbeispiele einer erfindungsgemäß ausgebildeten Gehörknöchelprothese anhand der beiliegenden schematischen Zeichnungen näher erläutert.

Im einzelnen zeigen:
- Fig. 1: eine schematisierte Seitenansicht einer Vollprothese;
- Fig. 2: eine Seitenansicht eines ersten Ausführungsbeispieles einer Teilprothese;
- Fig. 3: eine Seitenansicht einer zweiten Ausführungsform einer Teilprothese.

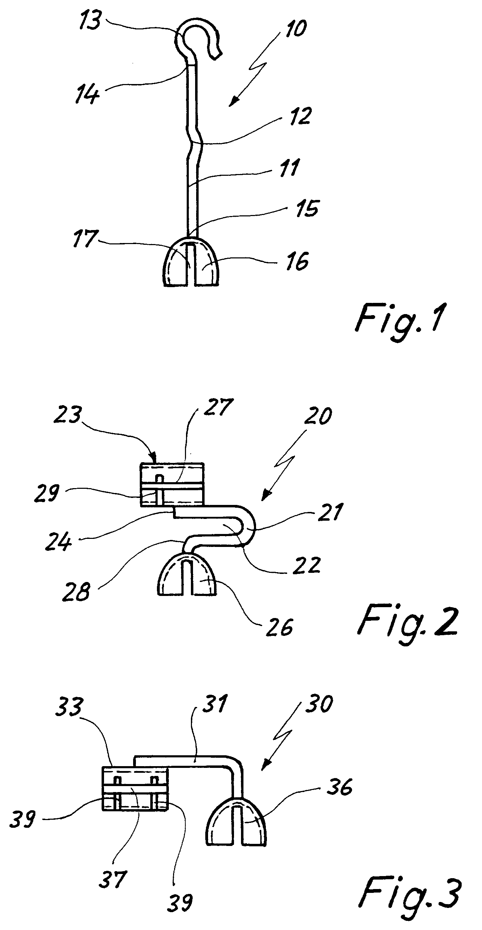

Die in Fig. 1 dargestellte Vollprothese 10 besteht aus einem Stiel 11, der aus einem Feingolddraht gehämmert ist und in seinem Mittelbereich eine eine Längenänderung erlaubende Auswölbung 12 aufweist. An dem in Fig. 1 oberen Ende des Stieles 11 ist ein aus reinem Titan gefertigter Hakenteil 13 mittels einer Laserschweißstelle 14 stumpf angesetzt. An dem anderen und in Fig. 1 unteren Ende ist der aus Feingold gefertigte Stiel 11 über eine Schweißstelle 15 mit einem Glockenkörper 16 verbunden. Dieser Glockenkörper ist zur Veränderung seines Durchmessers kreuzweise geschlitzt. In der Zeichnung ist einer der Kreuzschlitze 17, der senkrecht zur Zeichnungsebene verläuft, ersichtlich. Der andere Kreuzschlitz verläuft - aus der Zeichnung nicht ersichtlich - in der Zeichnungsebene. Der Hakenteil 13 dient zur Verankerung der Vollprothese 10 an einem Hammergriffrest am Trommelfell eines Patienten. Der Glockenkörper 16 dient zur Anlage der Vollprothese 10 gegen die Fußplatte eines Patienten bei nicht mehr vorhandenem Steigbügel und Amboß.

Die aus Fig. 2 ersichtliche Teilprothese 20 weist einen aus einem Feingolddraht gehämmerten Stiel 21 auf, der seitlich zu einem U ausgebogen ist. Am Ende des einen U-Schenkels ist über eine Schweißstelle 24 ein aus reinem Titan gefertigter Koppelungskörper in Form einer Manschette 23 befestigt. An dem anderen und in einer Abkröpfung 28 endenden U-Schenkel des Stieles 21 ist ein Glockenkörper 26 befestigt oder angeformt, dessen Form mindestens annähernd dem Glockenkörper 16 der Fig. 1 entspricht, hier jedoch ebenfalls aus Feingold gefertigt ist. Der geschlitzte Glockenkörper 26 ist zur Verankerung der Teilprothese 20 auf dem Steigbügelköpfchen bestimmt. Mit der Titanmanschette 23, die aus einer mit einem Längsschlitz 27 und einem zusätzlichen Teilquerschlitz 29 versehenen Titanbuchse besteht, erfolgt eine Verankerung der Teilprothese 20 auf dem Hammergriff.

Fig. 3 zeigt eine Teilprothese 30, bei welcher am einen Ende eines um 90° abgewinkelten und aus Feingold gefertigten Prothesenstieles 31 eine aus reinem Titan gefertigte Manschette 33 befestigt ist. Die Titanmanschette ist - wie die Manschette 23 nach Fig. 2 - aus einer Titanbuchse geformt, die mit einem durchgehenden Längsschlitz 37 und hier zusätzlich mit zwei Teilquerschlitzen 39 zur Erleichterung ihrer Verformung versehen ist. Die Teilquerschlitze 39 erstrecken sich etwa über 2/3 des Buchsenumfanges. An dem zweiten Ende des Stieles 31 ist ein hier wiederum aus Feingold gefertigter und kreuzgeschlitzter Glockenkörper 36 befestigt oder angeformt. Die Teilprothese 30 ist zur Verankerung mit ihrem Glockenkörper 36 auf einem Steigbügel und zur Verankerung mit ihrer Titanmanschette 33 auf einem Amboßschenkel vorgefertigt.

## Patentansprüche

1. Gehörknöchelprothese zur Schallübertragung im Mittelohr, mit einem aus Feingold gefertigten Stiel, dadurch gekennzeichnet, daß an mindestens einem der Enden des Stieles (11, 21, 31) ein aus reinem Titan gefertigter Koppelungskörper (13, 16, 23, 33) angeschlossen ist.

2. Gehörknöchelprothese nach Anspruch 1, als Vollprothese zwischen dem Trommelfell und der Fußplatte des Steigbügels, dadurch gekennzeichnet, daß sie am einen Ende des drahtförmigen Feingoldstieles (11) ein an einen Hammergriffrest am Trommelfell ansetzbares, aus reinem Titan gefertigtes Hakenteil (13) und am anderen Ende des Feingoldstieles (11) einen geschlitzten Glockenkörper (16) aus reinem Titan zur Anlage gegen die Fußplatte aufweist.

3. Gehörknöchelprothese nach Anspruch 1, als Teilprothese zwischen dem Steigbügel und dem Amboßschenkel oder dem Hammergriff, dadurch gekennzeichnet, daß sie am einen Ende des Feingoldstieles (21, 31) einen geschlitzten Glockenkörper (26, 36) aus Feingold zum Ansatz am Steigbügel und am anderen Ende des Feingoldstieles (21, 31) eine geschlitzte Manschette (23, 33) aus reinem Titan zum Ansatz am Amboßschenkel bzw. am Hammergriff aufweist.

4. Gehörknöchelprothese nach einem der Ansprüche 1 bis 3, dadurch gekennzeichnet, daß die Titanteile (13, 16, 23, 33) mit dem Feingoldstiel (11, 21, 31) durch Schweißen fest verbunden sind.

5. Gehörknöchelprothese nach einem der Ansprüche 1 bis 4, dadurch gekennzeichnet, daß der aus Feingold gefertigte Stiel (11, 21, 31) mindestens eine Abwinkelung, Ausbiegung (12) oder Abkröpfung (21, 28) aufweist.

6. Gehörknöchelprothese nach einem der Ansprüche 1 bis 5, dadurch gekennzeichnet, daß der am einen Ende des Stieles (11, 21, 31) angeordnete Glockenkörper (16, 26, 36) zur Veränderung des Glockendurchmessers kreuzweise geschlitzt ist.

7. Gehörknöchelprothese nach einem der Ansprüche 1 bis 6, dadurch gekennzeichnet, daß die aus Titan gefertigte, als Koppelungskörper dienende Manschette (23, 33) am einen Ende des Feingoldstieles (21, 31) aus einer längsgeschlitzten Titanbuchse besteht (Längsschlitz 27, 37), die zur Verbesserung ihrer Verformbarkeit an mindestens einer Stelle zusätzlich einen Teilquerschlitz (29, 39) aufweist.
